# EUROPEAN PATENT SPECIFICATION

(11) **EP 2 882 753 B1**
(45) Date of publication and mention of the grant of the patent: **05.10.2016**
(21) Application number: 13777144.0
(22) Date of filing: 08.08.2013
(51) Int. Cl.: C07D 487/04, A61K 31/519, A61P 35/00

(54) **PROCESS FOR THE PREPARATION OF PEMETREXED AND LYSIN SALT THEREOF**
VERFAHREN ZUR HERSTELLUNG VON PEMETREXED UND EINEM LYSINSALZ DAVON
PROCÉDÉ POUR LA PRÉPARATION DE PÉMÉTREXED ET DU SEL DE LYSINE DE CELUI-CI

(30) Priority: 08.08.2012 IT RM20120398
(43) Date of publication of application: 17.06.2015
(73) Proprietor: BERLIN-CHEMIE AG, 12489 Berlin (DE)
(72) Inventor: BONACCORSI, Fabrizio, I-56122 Pisa (IT); CALVANI, Federico, I-56122 Pisa (IT); PASQUI, Franco, I-56122 Pisa (IT)
(74) Representative: Germinario, Claudio
(86) International application number: PCT/IB2013/056497
(87) International publication number: WO 2014/024164

(56) References cited:
- EP-A1- 2 301 909
- EP-A2- 0 589 720
- EP-A2- 2 409 978
- WO-A1-2012/056285
- EDWARD C. TAYLOR ET AL: "A New and Efficient Synthesis of Pyrrolo 2,3- d |pyrimidine Anticancer Agents: Alimta (LY231514, MTA), Homo-Alimta, TNP-351, and Some Aryl 5-Substituted Pyrrolo 2,3- d |pyrimidines", THE JOURNAL OF ORGANIC CHEMISTRY, vol. 68, no. 26, 1 December 2003 (2003-12-01), pages 9938-9947, XP055002811, ISSN: 0022-3263, DOI: 10.1021/jo030248h cited in the application
- BARNETT C J ET AL: "A PRACTICAL SYNTHESIS OF MULTITARGETED ANTIFOLATE LY231514", ORGANIC PROCESS RESEARCH AND DEVELOPMENT, CAMBRIDGE, GB, vol. 3, no. 3, 1 January 1999 (1999-01-01) , pages 184-188, XP000982091, DOI: 10.1021/OP9802172 cited in the application
- MIWA TETSUO ET AL: "A novel synthetic approach to pyrrolo(2,3-d)pyrimidine antifolates", JOURNAL OF ORGANIC CHEMISTRY, ACS, US, vol. 58, no. 7, 1 January 1993 (1993-01-01), pages 1696-1701, XP002482880, ISSN: 0022-3263, DOI: 10.1021/JO00059A016 cited in the application

## Description

The present invention refers to a process for the preparation of pemetrexed and salts thereof, in particular a lysine salt thereof, to said salt as such and to pharmaceutical compositions comprising the same.

### BACKGROUND OF THE INVENTION

Pemetrexed is normally used in pharmaceutical preparations in the form of disodium salt, specifically the disodium heptahydrate salt is the product marketed by Eli Lilly and Company under the trademark ALIMTA® and it is described in EP1259513.

Pemetrexed was originally described in EP432677/US5344932. The synthesis shown in this patent provides the 2-amino-7H-pyrrolo (2,3-d) pyrimidin-4-ol as a raw material, the amino group of this derivative is protected with pivaloyl chloride and the product obtained is transformed in two steps in the corresponding 3-iodo derivative. The subsequent coupling reaction with dimethyl N-(4-etinilbenzoil)-L-glutamate, provides a derivative biarilacetylenic that is converted to pemetrexed by catalytic hydrogenation and subsequent saponification. This procedure, however, is not suitable for industrial application.

The pemetrexed diacid may also be obtained by following other most recent synthetic procedures among these those reported in U.S. 5,416,211, in EP 9916742 and in 905128/WO CJ Barnett et al., Org. Proc Res & Develop., 1999, 3, 184-188, using industrial syntheses that require the preparation of the intermediate pemetrexed diethyl ester 3 (Scheme I):

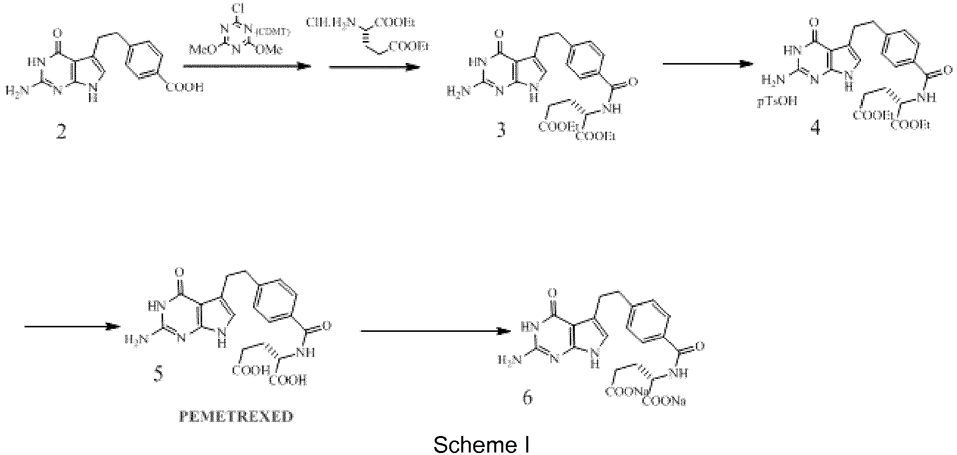

In the references cited pemetrexed diethyl ester 4 is prepared by reacting

the acid 4-(2-(2-amino-4,7-dihydro-4-oxo-3H-pyrrolo (2,3-d) pyrimidin-5-yl) ethyl) benzoic, (2), with the L diethyl hydrochloride glutamate in the presence of a condensing agent in N, N-dimethylformamide as solvent. This procedure does not provide for the isolation of pemetrexed diethyl ester (3): after the extractive work-up with dichloromethane, and solvent exchange with ethanol, the aforementioned product is isolated and purified as a salt of acid p-toluenesulfonic (4) which is subsequently converted in pemetrexed (5) by base hydrolysis and acidification. The diacid pemetrexed (5) is converted to the corresponding disodium salt by treatment with aqueous soda.

This process involves the use of DMF (a solvent with high toxicity) as a solvent of the coupling reaction, and also, even it allows to obtain the pemetrexed with high purity, consists of a long sequence of operations to isolate and purify the pemetrexed diester salt (4) namely, 3 grindings and a crystallization with relative filtrations, consequently reducing the efficiency of the process.

A further negative aspect of the purification procedure of ester pemetrexed (3) through the formation of p-toluenesulphonic acid salt is represented by the possible formation of esters by the reaction of p-toluenesulfonic acid with alcohols. Such esters p-toluensulphoonic products are classified as genotoxic alkylating agents and their content must be reduced to very low values (Genotoxic and carcinogenic impurities in drug substances and Products: Recommended Approaches, FDA Guidance for Industry, 2008; Guideline on the Limits of genotoxic Impurities, EMEA/CHMP 2006).

The pemetrexed diethyl ester (3) is however described in the literature as solid compound: in CJ Barnett et al, Org. Proc Res & Develop., 1999, 3, 184-188, on page 188 the compound 13 is shown with a pf of 169-171 °C; in E.C. Taylor and B.Liu, J.Org. Chem., 2003, 68, 9938-9947, on page 9945 the compound 25a is shown with a pf 84-86°C; in T.Miwa et al, J.Org. Chem., 1993, 58, 1696-1701, at page 1700, the compound 23a is shown with a pf 131-133°C.

In EP 2409978 is claimed a new process for preparing the pemetrexed diester (3) that allows to eliminate the procedure of synthesis and purification of (4). In particular, the coupling reaction between the acid (2) and diethyl glutamate is carried out in an aprotic organic solvent, in particular in a mixture of DMF and dichloromethane; at the end of the reaction, the mixture is washed with a basic aqueous solution, the solution is concentrated, added with a polar organic solvent, preferably ethanol and pemetrexed diester (3) is isolated by crystallization. Even in this process are employed solvents with high toxicity (DMF and dichloromethane), and also the work-up of the reaction mixture after the coupling necessarily involves a high volume of solvents and numerous washes of the organic phase with aqueous solutions to remove the DMF present, consequently limiting the efficiency and productivity of the process. Pemetrexed disodium salt (6) is then obtained by saponification of the ester (4).

Pemetrexed disodium salt employed in pharmaceutical formulations is characterized by a poor stability and shows the formation of degradation impurities, both in the solid state and in solution, in particular related to the effects of oxidative type, which require suitable conditions of handling and packaging in a controlled atmosphere and preclude the use of liquid formulations ready for use. Recently, in WO 2012/015810, aqueous formulations of pemetrexed salts with antioxidants and chelating agents which have greater long-term stability have been reported, but however they show an increase of the degradation products up to values of 1-5%, which is an impurities amount not acceptable on the basis of the ICH guidelines of pharmaceutical products, if not properly qualified.

Scope of the present invention is to overcome the problems present in the state of the art and refer to the synthesis of pemetrexed and the instability of disodium salt thereof.

### SUMMARY OF THE INVENTION

The present invention is based on the discovery that the use of water-alcohol mixtures as a solvent in the coupling reaction (coupling) of the synthesis of pemetrexed allows the precipitation of the intermediate diethyl ester directly in the reaction mixture, without the need of DMF. The pemetrexed diethyl ester thus obtained has already a high purity that allows directly its use in subsequent steps of the synthesis of pemetrexed without the need to perform steps of salification, purification and / or washes as is described in the state of the art according to the methods in which DMF is used.

Furthermore, the inventors of the present invention have observed that the pemetrexed (bi)lysine salt is surprisingly and unexpectedly more stable than the already known disodium salt. This greater stability of the bilysine salt involves a considerable technical advantage since they overcome the problems of instability of the active ingredient pemetrexed in handling and storage steps.

For the purposes of the present invention the pemetrexed bilysine salt may be generically referred to as lysine salt as well as the disodium salt may be generically referred to as the sodium salt.

Therefore, objects of the present application are:
- a process for the preparation of a pemetrexed salt of general formula I 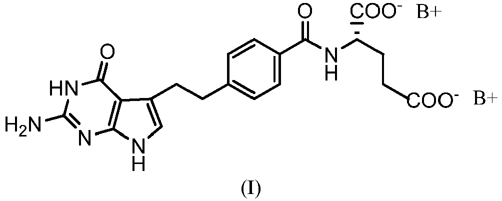 in which B + is a suitable cation, preferably Na+ or protonated lysine, comprising the following steps:
   a) reacting the acid 4-(2-(2-amino-4 ,7-dihydro-4-oxo-3H-pyrrolo (2,3-d) pyrimidin-5-yl) ethyl) benzoic (2) with the L -diethyl glutamate hydrochloride in the presence of a coupling reagent to obtain the pemetrexed diethyl ester (3) 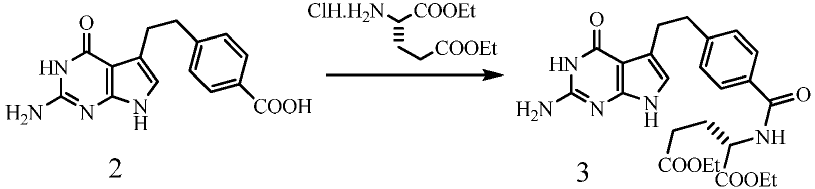
   b) hydrolyzing the pemetrexed diethyl ester (3) to acid pemetrexed (5) 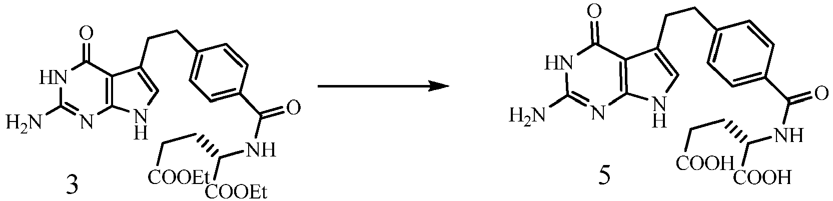
   c) salifying with a base selected from NaOH or lysine to obtain the corresponding pemetrexed salt
   characterized in that in step a):
   1) the acid (2) is previously converted *in situ* to the corresponding sodium carboxylate or morpholine carboxylate which may be isolated or directly reacted;
   2) the reaction solvent used consists of a hydro-alcoholic mixture of solvents wherein the alcohol is selected from the group: methanol, ethanol, isopropanol, n-propanol;
   3) the intermediate pemetrexed diethyl ester (3) directly precipitates from the reaction mixture with a purity >98%.

A further object of the present invention is an acid-N-[4-[2-(2-amino-4-oxo-4,7-dihydro-3H-pyrrolo [2,3-d] pyrimidin-5-yl) ethyl] benzoyl]-L-glutamic (pemetrexed) salt of (bi)lysine of formula (1) 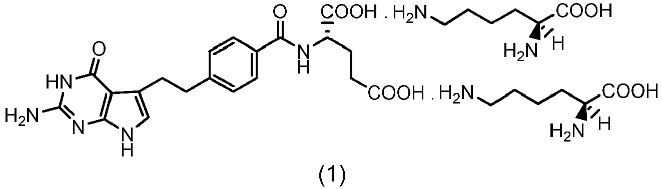 in an anhydrous or hydrate form, preferably in its crystalline form defined by the diffraction X-ray spectrum shown in Figure VII.

Further objects of the application are the compound according to formula (1) above for use as a medicament, preferably as anti-tumour agent;
a process for the synthesis of the compound of formula (1) comprising a step of salification of pemetrexed biacid (5) with the lysine base;
- a pharmaceutical composition comprising the compound according to formula (1) and/or a crystalline form thereof as above indicated and at least one pharmacologically acceptable excipient.

### DRAWINGS

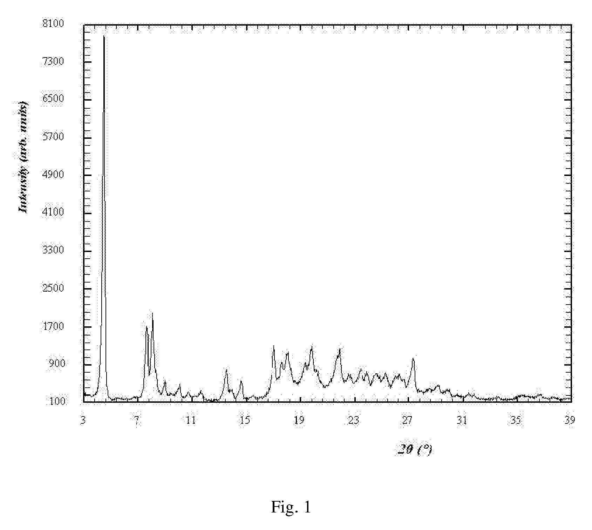

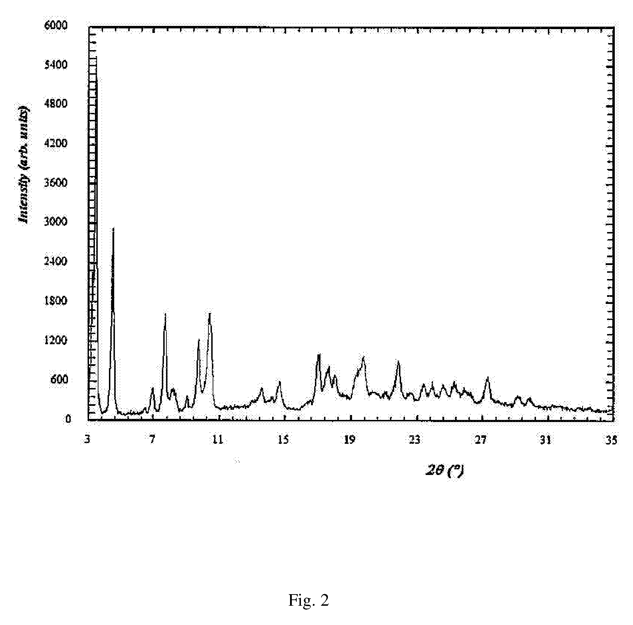

Figure 1 shows the X-ray diffraction spectrum of diethyl ester pemetrexed precipitated from isopropanol/water.
Figure II shows the X-ray diffraction spectrum of diethyl ester pemetrexed precipitated from ethanol/water/acetone.
Figure III the DSC (differential scanning calorimetry) profile of pemetrexed diethyl ester precipitated from ethanol/water/acetone.
Figure IV shows the X-ray diffraction spectrum of pemetrexed diethyl ester crystallized from 2-methyl tetrahydrofuran / ethanol.
Figure V shows the the X-ray diffraction spectrum of pemetrexed diethyl ester crystallized from ethanol/methyl ethyl ketone.
Figure VI the DSC (differential scanning calorimetry) profile of pemetrexed diethyl ester crystallized from ethanol/methyl ethyl ketone.
Figure VII shows the X-ray diffraction spectrum of pemetrexed lysine salt.
Figure VIII shows the data of X-ray diffraction of pemetrexed lysine salt.
Figure IX shows the DSC (differential scanning calorimetry) profile of the lysine salt.

### DETAILED DESCRIPTION OF THE INVENTION

The present invention consists of the following aspects, which are described in detail below.

### Process of synthesis

The process of synthesis object of the present description is a process for the preparation of a pemetrexed salt, of general formula I 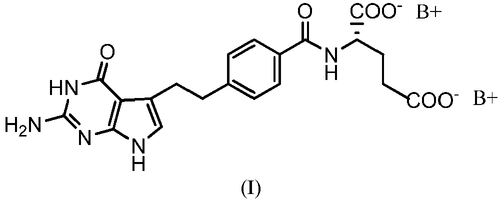 in which B + is a suitable cation, preferably selected from Na+ or protonated lysine.

This salt is obtained by a new process of synthesis of industrial applicability (scheme II) that allows to obtain easily the intermediate of synthesis (3), the pemetrexed ethyl diester, in solid state avoiding the use of DMF.

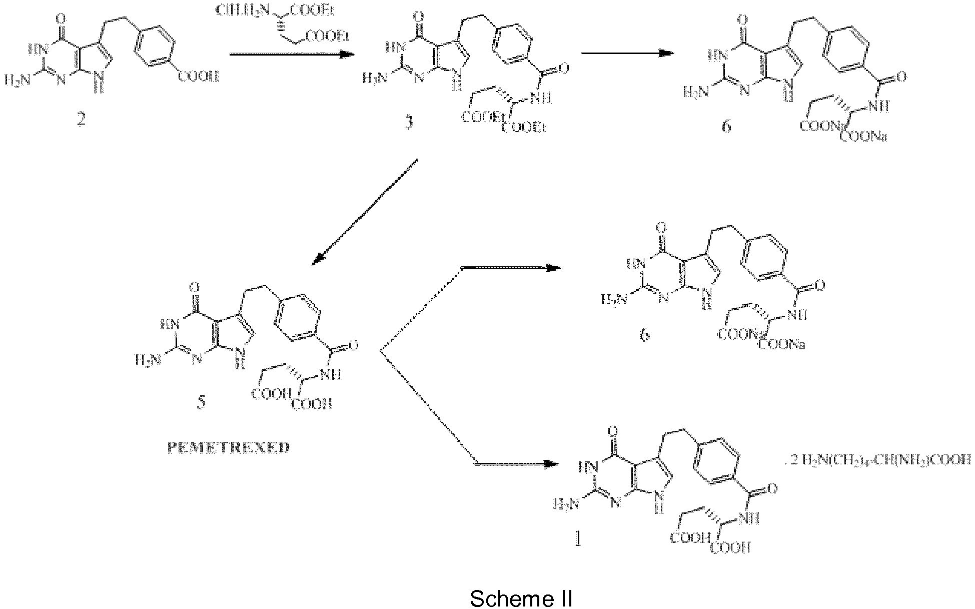

In particular, the use of hydroalcoholic mixtures (preferably isopropanol/water) as the solvent of the coupling reaction (coupling) results in the precipitation of the intermediate (3) from the reaction mixture. The pemetrexed diester (3) obtained after filtration has already a high purity (≥ 98%) that allows the direct use of it in subsequent steps without further purification.

The pemetrexed diester (3) thus prepared can be converted directly into the desired pemetrexed salt, for example the disodium salt of dilysine or, without the need to isolate the pemetrexed in free acid form that is essential when preparing the pemetrexed ester salt with p-toluene sulfonic acid, as described in the state of the art. The direct formation of the pemetrexed salt without the need to isolate the pemetrexed in acid form represents a further advantage of the process of preparation object of the present invention.

The pharmaceutically acceptable pemetrexed salts obtainable with the process object of the present invention are, in particular, without being limited to, the disodium salt and the (bi)lysine salt.

These salts may be either in anhydrous form or in hydrated form. Preferably, said salt comprises a percentage of water comprises in the range 0-22% by weight. It had to be considered for example that the compound in the market, Alimta®, corresponds to the heptahydrate disodium pemetrexed and contains a percentage of water equal to 21% by weight, and another pemetrexed salt well known literature, the hemipentahydrate disodium pemetrexed has instead a percentage of water equal to 8.7% by weight.

The process for the preparation of the salt of formula 1, according to the present invention comprises, in particular, the following steps:
a) reacting the acid 4-(2-(2-amino-4,7-dihydro-4-oxo-3H-pyrrol(2,3-d) pyrimidin-5-yl) ethyl) benzoic (2) with the L -diethyl glutamate hydrochloride in presence of a coupling reagent to obtain the pemetrexed diethyl ester (3);
b) hydrolysing the pemetrexed diethyl ester (3) to pemetrexed acid (5)
c) salifying with a base selected from NaOH or lysine to obtain the corresponding pemetrexed salt.

For the synthesis of the acid 4-(2-(2-amino-4 ,7-dihydro-4-oxo-3H-pyrrolo (2,3-d) pyrimidin-5-yl) ethyl) benzoic, (2) numerous synthetic procedures are reported in the literature among which we can cite EP432677, EP 905 128, US 5,416,211, EP 2301909, CJ Barnett et al., Org. Proc Res & Dev., 1999, 3, 184-188. In general the acid 2 can be conveniently prepared by following the general synthetic procedure described in the following Scheme III:

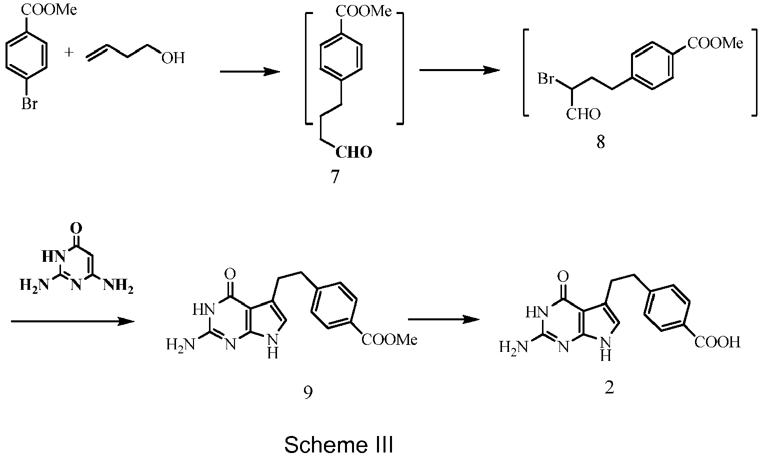

In EP 905 128 (Example 1, p 10) the crude aldehyde 7 (yield 87%) is not isolated and purified, but the organic solution containing the aldehyde 7 obtained is filtered on a resin to remove the elementary Pd still present, and organic solvent by dry evaporation. The crude aldehyde 7 thus obtained is then purified by treatment with sodium bisulfite and formation of the corresponding derivative sulfonic acid sodium salt (derivative also known as 'Bertagnini's compound') in the form of a solid crystalline (EP905128, Example 2, page 11).

In EP 2301909 it is reported instead that it is not necessary to purify the crude aldehyde 7 by means of the derivative sulfonic acid sodium salt, but it would be sufficient an extraction with organic solvent (preferably a mixture of solvents) of the reaction mixture, a discoloration on silica gel and the subsequent dry evaporation of the organic solvent to obtain an aldehyde (7) already with a purity of 96% suitable for the subsequent synthetic steps.

We have now found that instead it is sufficient to operate as described in EP905128, example 1, to obtain a solution in ethyl acetate of the crude aldehyde 7, which can be directly used in the subsequent step of bromination, without any need for purification by the salt sodium sulfonic derivative, as expected from EP905128, or extraction, isolation and discoloration, as expected from EP2301909.

The crude aldehyde (7) is transformed into the corresponding brome aldehyde which in turn is reacted directly with 2,4-diamino-6-hydroxy pyrimidine, so obtaining the methyl 4-(2-(2-amino-4,7-dihydro-4-oxo-1H-pyrrolo(2,3-d)pyrimidin-5-yl)ethyl)benzoate (9) (EP905128, example 4, page 11).

The saponification of the ester (9) and the subsequent acidification of the reaction mixture leads to the carboxylic acid (2) (EP905128, example 5, page 11) having the suitable purity for the preparation of disodium pemetrexed for pharmaceutical uses.

The process of pemetrexed synthesis object of the present application is characterized mainly by the fact that the reaction solvent used in the synthetic step a) of Scheme I is constituted by a mixture of water and ethanol solvents in which the alcohol is selected from the group methanol, ethanol, isopropanol, n-propanol. Preferably the solvent mixture is represented by isopropanol/water or water/ethanol. In particular, the water and isopropyl alcohol or ethyl alcohol are in ratio v/v comprised between 3/1 and 1/3, preferably between 6/4 and 4/6. The amount of the solvent mixture used is between 5 and 25 volumes with respect to the acid precursor (2) and preferably between 10 and 20 volumes, meaning by the term "volume" the ratio solvent/solute expressed in ml of solvent/g of precursor.

Under the reaction conditions in the synthetic step a) the intermediate pemetrexed diethyl ester (3) precipitates directly from the reaction mixture with a purity>98% and can be possibly used directly in the next step without further purification.

Moreover, in the synthetic step a), the acid (2) is previously converted *in situ* into the corresponding sodium carboxylate which can be isolated (US2008/0146799, example 5, p 13) or directly reacted. Alternatively, always in the synthetic step a), the acid 2 can be converted *in situ* into the corresponding morpholinium carboxylate which is then reacted directly. The salification *in situ* can be performed according to the usual methods which are known to the skilled person.

The coupling reagent is selected from CDMT (2-chloro-4 ,6-dimethoxy-1,3,5-triazine), EDC (1-ethyl-3 (3-dimethylaminopropyl) carbodiimide, DMTMM (4-(4,6-dimethoxy-1,3,5-triazin-2-yl)-4-methylmorpholine chloride) and more preferably CDMT (2-chloro-4 ,6-dimethoxy-1,3,5-triazine).

The reaction takes place at a temperature between 0°C and 60°C, preferably between 15 and 50°C.

The complete precipitation of pemetrexed diethyl ester (3) is obtained by further addition to the reaction mixture, of 5-35 volumes, preferably 10-25 volumes, of water or a water/acetone mixture in a ratio comprises from 2/1 to 1/1.

The suspension obtained can be maintained under stirring at temperatures between 0 and 5°C, preferably between 5 and 30°C, for a time between 1 and 24 hours, preferably between 5 and 12 hours.

The diester pemetrexed (3) thus obtained, after filtration and washing with a hydro alcoholic solvents mixture, can be subsequently dried under vacuum at about 50°C up to a constant weight. This crude product exhibits high HPLC purity >98% and, for the purposes of the present invention, can be used directly without further purifications in the subsequent reaction of hydrolysis to the free acid.

Then, the conversion of diester pemetrexed (3) to acid (5) can be carried out according to the process already described in US 5,416,211 or EP 905128 by hydrolysis in aqueous solution in the presence of a base. Pemetrexed is then isolated after acidification, by precipitation from water-alcohol mixtures.

The reaction temperature is between 0 and 40°C, preferably between 10 and 30°C.

In the formation of the pemetrexed salt, the acid (5) is salified with a suitable base, preferably selected from NaOH or lysine to obtain the corresponding pemetrexed salt.

The process described here can also be used to prepare the pemetrexed disodium salt that may be obtained from the biacid precursor (5), for example, by treatment with sodium hydroxide in aqueous solution up to pH 8 and precipitation with a water miscible solvent in analogy to what is reported in EP 905 128.

Alternatively, the high purity of diester pemetrexed (3) allows to obtain the disodium salt in a single step directly by basic hydrolysis in a hydro alcoholic solution of (3) and precipitation of the disodium salt by addition of a water miscible solvent.

Diester pemetrexed of formula 3 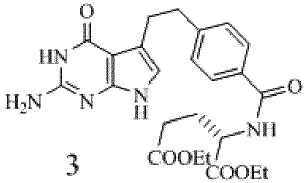 obtained according to the process described above has a predominantly amorphous form whose degree of crystallinity may vary depending on the batches of reaction (Figure 1).

The crude diester pemetrexed can be used without further purification in subsequent reactions or may be further purified, for example, for a better characterization, by crystallization using a series of solvents selected from acetonitrile, ethyl acetate / ethanol, 2-methyl tetrahydrofuran/ethanol, methyl ethyl ketone/ethanol, isopropanol/water, yielding a pemetrexed diethyl ester (3) with a purity > 99.5%, as shown by HPLC.

In particular, the authors of the present invention have observed that: using as the mixture of crystallization 2-methyl tetrahydrofuran/ethanol a crystalline solid of pemetrexed diester (3) exhibiting a spectrum of X-ray diffraction shown in Figure IV, is obtained while using as the mixture of crystallization ethanol/methyl ethyl ketone a crystalline solid of pemetrexed diester (3) exhibiting a spectrum of X-ray diffraction shown in Figure V is obtained.

Pemetrexed Lysine salt.

Object of the present invention is also the acid compound N-[4-[2-(2-amino-4-oxo-4 ,7-dihydro-3H-pyrrolo [2,3-d] pyrimidin-5-yl) ethyl] benzoyl]-L-glutamic (pemetrexed) lysine salt of formula (1) 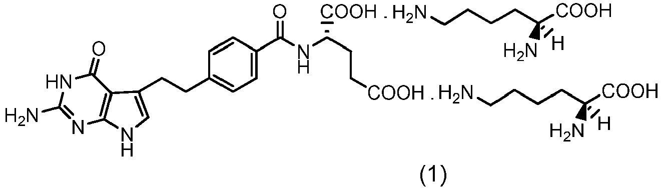 in anhydrous or hydrated form.

Preferably, this salt has a percentage of water comprises in the range 0-22% by weight.

Furthermore, the lysine salt can be obtained as crystalline solid and is characterized by X-ray diffraction spectrum shown in Figure VII, and summarized in Figure VIII.

The lysine salt is also characterized by a profile DSC (differential scanning calorimetry) shown in Figure IX, wherein has a melting point at 230°C followed by decomposition. The thermogravimetric analysis (TGA) shows a weight loss of 3.8%.

The pemetrexed lysine salt object of the present application is characterized by a significantly higher stability compared to the disodium salt as shown in the following tables.

**Table 1: Results of HPLC purity (area%) for pemetrexed hemipentahydrate disodium salt stored at 25°C ± 5°C and 40°C ± 5°C (humidity not controlled)**

| | | 25°C±5°C | | | 40°C±5°C | | |
|---|---|---|---|---|---|---|---|
| | | months | | | months | | |
| Related substances (R_{R}) | 0 | 1 | 3 | 6 | 1 | 3 | 6 |
| 0.19 | n.r. | n.r. | n.r. | 0.04 | n.r. | 0.50 | 0.40 |
| 0.26 | n.r. | n.r. | n.r. | 0.31 | n.r. | 0.17 | 0.98 |
| Pemetrexed | 99.78 | 99.71 | 99.78 | 99.47 | 99.66 | 99.00 | 98.00 |
| 1.15 | n.r. | n.r. | 0.02 | n.r. | 0.03 | 0.11 | 0.10 |
| Other (max) | 0.06 | 0.07 | 0.07 | 0.06 | 0.05 | 0.06 | 0.20 |

**Table 2: Results of HPLC purity (area%) for pemetrexed lysine salt stored at 25 ° C ± 5 ° C and 40 ° C ± 5 ° C (humidity not controlled).**

| | | 25°C±5°C | | | 40°C±5°C | | |
|---|---|---|---|---|---|---|---|
| | | months | | | months | | |
| Related Substances (R_{R}) | 0 | 1 | 3 | 6 | 1 | 3 | 6 |
| 0.19 | n.r. | n.r. | 0.07 | 0.05 | n.r. | 0.14 | 0.08 |
| 0.26 | n.r. | n.r. | n.r. | n.r. | n.r. | n.r. | n.r. |
| 0.44 | 0.03 | 0.03 | 0.04 | 0.04 | 0.04 | 0.05 | 0.06 |
| Pemetrexed | 99.83 | 99.82 | 99.74 | 99.72 | 99.79 | 99.61 | 99.58 |
| 1.15 | 0.04 | 0.04 | 0.07 | 0.04 | 0.04 | 0.09 | 0.08 |
| Other (max) | 0.04 | 0.05 | 0.04 | 0.08 | 0.04 | 0.05 | 0.08 |

**Table 3: Results of the HPLC purity (area %) for the pemetrexed hemipentahydrate disodium salt, heptahydrate and lysine salt stored at 100°C for 6 days (humidity not controlled)**

| | Pemetrexed hemipentahydrate disodium salt | | Pemetrexed heptahydrate disodium salt | | Pemetrexed lysine salt | |
|---|---|---|---|---|---|---|
| | days | | days | | days | |
| Related Substances (R_{R}) | 0 | 6 | 0 | 6 | 0 | 6 |
| 0.19 | n.r. | 0.38 | n.r. | 1.01 | 0.07 | 0.13 |
| 0.26 | n.r. | 2.08 | n.r. | 0.46 | n.r. | n.r. |
| 0.44 | n.r. | 0.06 | n.r. | 0.12 | 0.09 | 0.22 |
| Pemetrexed | 99.88 | 96.72 | 99.95 | 97.36 | 99.67 | 99.41 |
| 1.15 | n.r. | 0.54 | n.r. | 0.55 | 0.11 | 0.10 |
| Others (max) | 0.06 | 0.06 | n.r. | 0.30 | n.r. | n.r. |

The pemetrexed disodium salt shows a significant degradation in accelerated conditions at 40°C, and the formation of 3 main impurities in relative retention times of 0:19, 0:26 and 1:15 has already observed after 3 months. In the same conditions the lysine salt is practically unaltered.

A study in stress conditions of 100°C for 6 days shows a remarkable increase of impurities in retention times of 0:19, 0:26 and 1:15 and both in the case of the hemipentahydrate disodium salt (0.4% -2%) and commercial heptahydrate disodium salt (0.4-1%).

The main degradation products were identified by HPLC/MS and these structures appear to be attributable to oxidation of the product. A similar pattern of impurities is obtained by treatment of pemetrexed disodium salt in aqueous solution in the presence of oxidizing agents such as hydrogen peroxide 10%.

For further confirmation of the oxidative type degradation, a sample of disodium salt packaged in a controlled atmosphere under nitrogen, under stress conditions of 100°C for 6 days shows a remarkable reduction in the impurities content.

The lysine salt is also much more stable under stress conditions of 100°C for 6 days and the same impurities do not exceed the value of 0.2%.

Therefore these data demonstrate how the pametrexed lysine salt is significantly more stable than the corresponding disodium salt and therefore can be advantageously used as a salt of the active ingredient pemetrexed since it is less prone to degradation/decomposition caused by oxidative stress.

The pemetrexed lysine salt, as also its crystalline form defined by x-ray diffraction spectrum of Figure VII, is advantageously usable as a medicament and preferably, similarly to the pemetrexed disodium salt, as anti-tumour drug.

The pemetrexed lysine salt can be obtained in general through a passage of salification of pemetrexed biacid (5) with the base lysine. In particular, in one embodiment of the method the salt of formula I can be obtained by adding to an aqueous solution of pemetrexed acid (5) from 2 to 4 equivalents of lysine hydrate, preferably 3 equivalents. Subsequently, the lysine salt formed is precipitated from the reaction mixture by addition of a water miscible solvent such as acetone, ethanol, methanol or isopropanol, preferably ethanol.

The volumes of water are between 3 and 8 with respect to the acid precursor and preferably 5 volumes, the volumes of the organic solvent are between 18 e 48 volumes compared to the acid precursor, preferably 30 volumes meaning with the term "volume" the ratio solvent/solute in ml of solvent/g of precursor.

### Pharmaceutical compositions

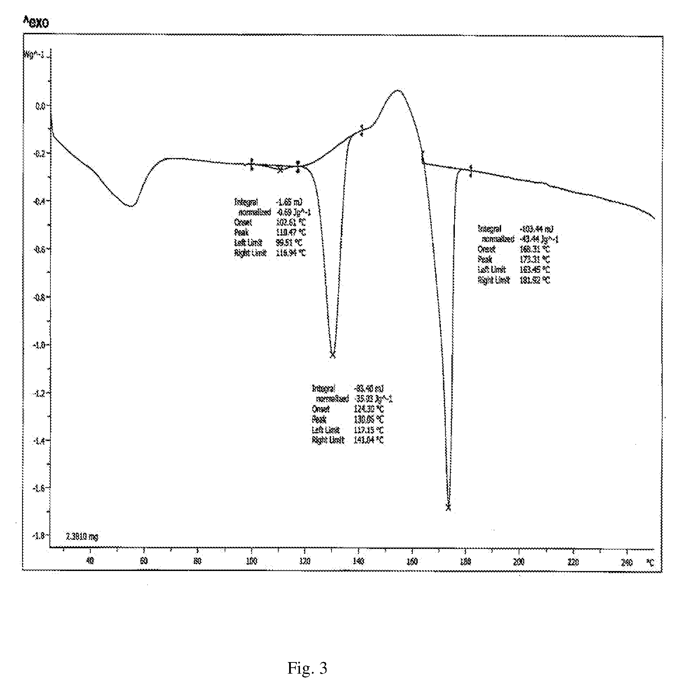

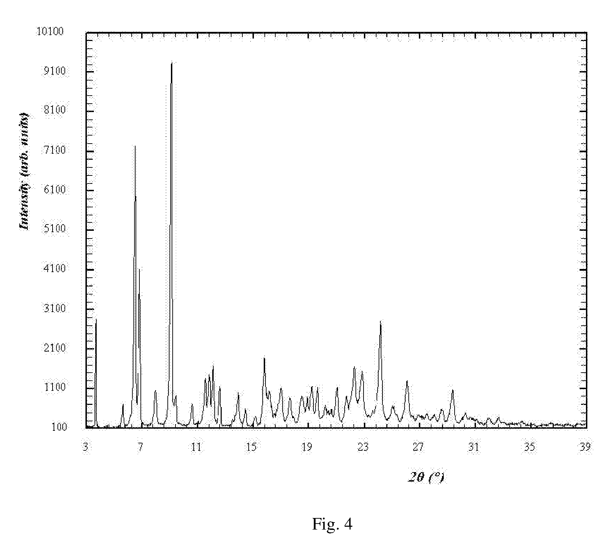

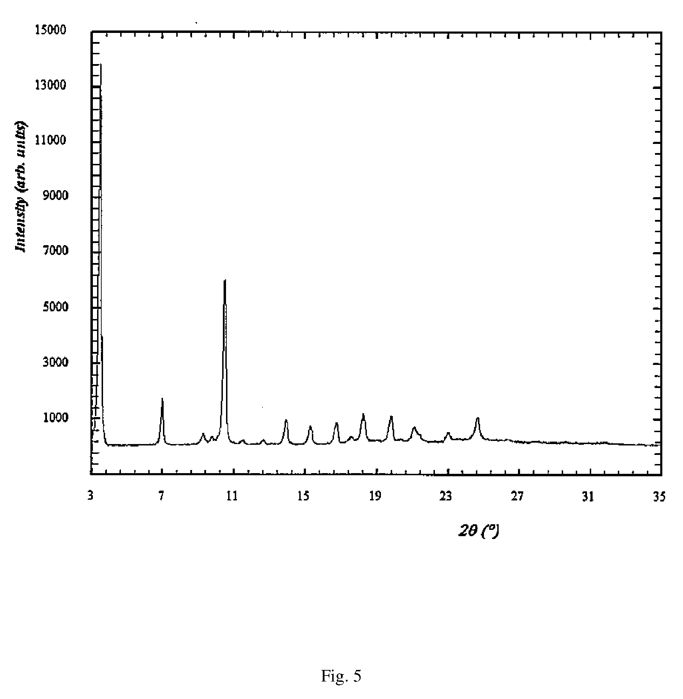

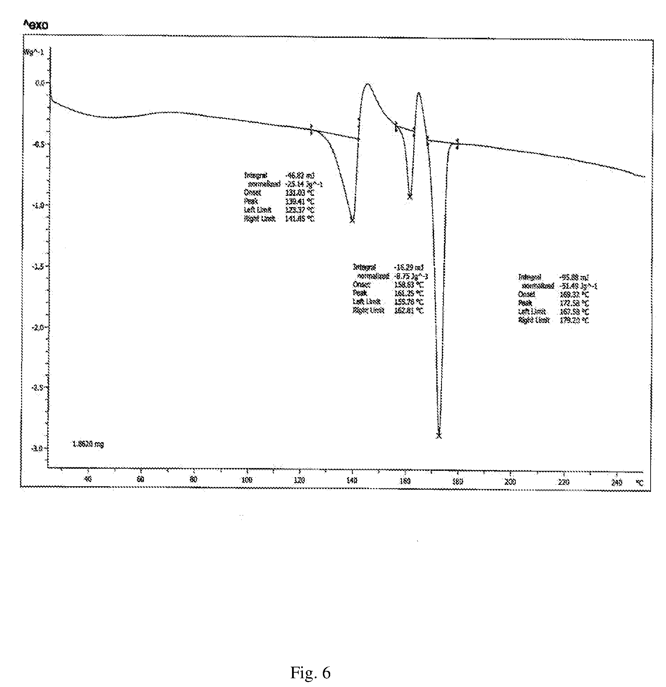

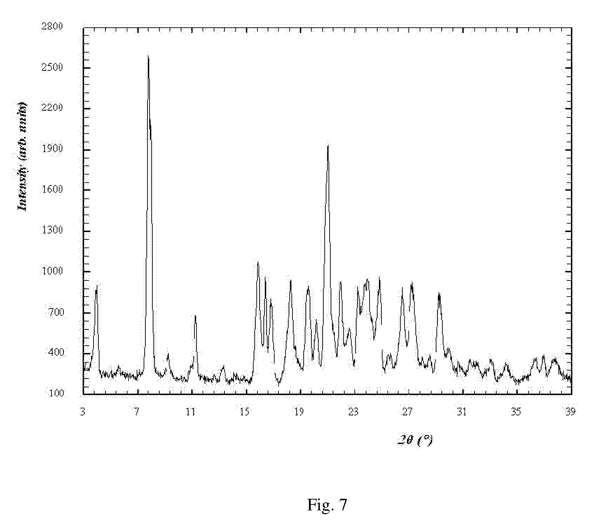

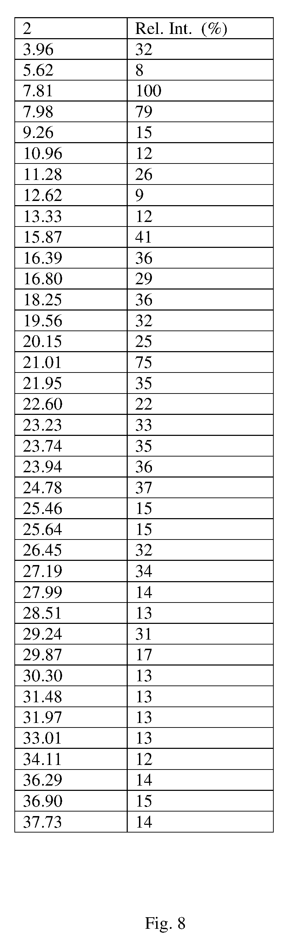

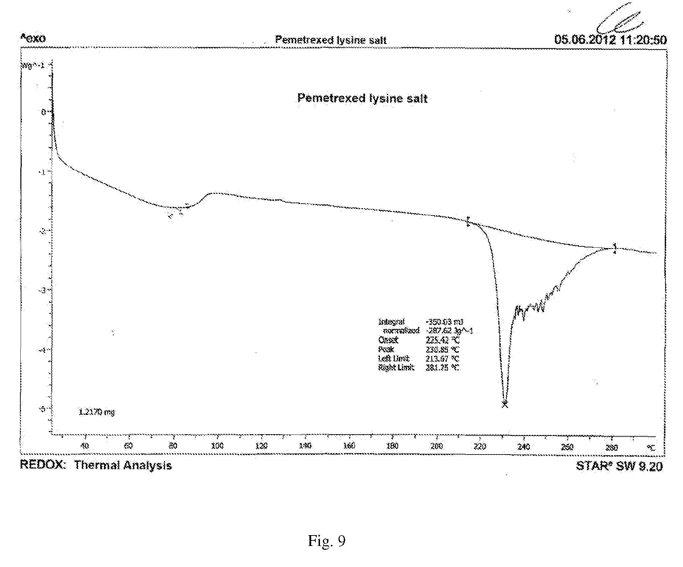

The pharmaceutical compositions of the present invention are compositions/formulations comprising the pemetrexed lysine salt of formula I or its crystalline form defined by the spectrum of Figure 3 and one or more pharmaceutically acceptable excipients. Said compositions are primarily intended for use as anti-tumour.

The compositions described herein are preferably administered by oral, parenteral or intravenous route and, in any case, according to all the routes of administration known to those skilled in the art and suitable to achieve the target site of pharmacological interest.

The compositions for oral administration may take the form of liquid solutions or suspensions or powders. The compositions may be presented in unit dosage form in order to facilitate the administration.

Each unit dose contains a predetermined amount of active ingredient calculated to produce the desired therapeutic effect, in association with a suitable pharmaceutical excipient. Typical unit dosage forms include pills, tablets, capsules or the like in the case of solid compositions and vials or syringes in the case of liquid compositions.

In such solid forms the active ingredient(s) is mixed with at least one inert pharmaceutically acceptable excipient or carrier and usual additives.

The compositions for parenteral use may be, for example, solutions, aqueous or oil suspensions or emulsions and can be formulated according to the known art using suitable dispersing agents, wetting agents and suspending agents.

They may also be present preservatives and other additives such as antimicrobial, anti-oxidants, chelating agents and inert gases (Mack (1982) Remington's Pharmaceutical Sciences, 16th Edition).

The components comprised in the pharmaceutical compositions of the present invention may also be lyophilized for storage and reconstituted in a suitable carrier prior to use.

A typical example of a pharmaceutical composition according to the present invention_can be so represented:

| | |
|---|---|
| Pemetrexed disodium or lysine salt: | 25 mg/ml (as pemetrexed acid) |
| Mannitol : | 25 mg/ml |
| HCl | up to pH = 7.0 - 7.4 |
| NaOH | up to pH = 7.0 - 7.4 |
| Water | q.s. |

A quantity of solution countering the desired amount of medicament is distributed in vials and lyophilized.

### EXAMPLES

The following are non-limiting embodiments of the present invention.

### EXAMPLE 1

### Preparation of methyl 4-(4-oxobutil)benzoate, (4-(4-carbometoxyphenil) butanal), compound (7) of Scheme III.

13.0 g of lithium acetate dehydrate, 14.7 g of lithium chloride and 17.1 g of tetra butyl ammonium chloride are added to a solution of 25.0 g of methyl p-bromo benzoate in 285 ml of DMF. Nitrogen is bubbled into the mixture and then adding 12 ml of 3-buten-1-ol and 0.52 g of Palladium Acetate. The mixture, heated to 75°C, allowed to react until complete conversion of the methyl p-bromo benzoate (HPLC control).

After cooling to room temperature to the reaction mixture 285 ml of water and 285 ml of ethyl acetate are added. After separation of the phases, the aqueous phase is extracted with ethyl acetate (2 x 143 ml), the combined organic phases are washed with 3% NaCl (3 x 285 ml) and with water (2 x 285 ml). The product (7) is obtained in solution of ethyl acetate with a yield equal to about 85% and is used as such, without isolation or further purification, in the next reaction.

### EXAMPLE 2

### Preparation of methyl (RS)-4-(3-bromo-4-oxo butyl) benzoate, compound (8) of Scheme III.

125 ml of the solution in ethyl acetate from Example 1, containing 3 g of (7), are concentrated under vacuum to about 22 ml. The resulting solution is cooled to 0°C and added with 0.126 ml of 33% HBr in AcOH and 0.97 ml of bromine. After 1 hour of stirring at 0°C 2% sodium metabisulphite (21 ml) are added to the reaction mixture, the separated organic phase is washed with saturated sodium bicarbonate (21 ml) and with 3% NaCl (21 ml). The solution is dry-evaporated at reduced pressure, the residue solubilized in 46 ml of acetonitrile and the resulting solution is used directly in the next step.

### EXAMPLE 3

### Preparation of methyl 4-(2-(2-amino-4,7-dihydro-4-oxo-3H-pyrrolo(2,3-d) pyrimidin-5-yl) ethyl) benzoate, compound (9) of Scheme III.

46 ml of water, 6.2 g of 2,4-diamine-6-hydroxypirimidine and 2.72 g of sodium acetate are added to the solution of bromo-derivative (8) in acetonitrile of Example 2. The mixture is stirred at 40°C for about 3.5 hours, cooled to room temperature and filtered. The solid is washed with 35 ml of acetonitrile/water 1/1 and dried under vacuum at 50°C. 3.4 g of (9) in the form of pale pink solid (combined yield with respect to (7) equal to 69%) were obtained.

### EXAMPLE 4

### Preparation acid 4-(2-(2-amino-4,7-dihydro-4-oxo-3H-pyrrolo(2,3-d) pyrimidin-5-yl) ethyl) benzoic, compound (2) of Scheme I.

A suspension of 14.0 g of (9) from the example 3 in 162 ml of 2 M NaOH was heated at 40° C for 1 hour. After cooling at room temperature the reaction mixture is diluted with 170 ml of acetone and the pH is adjusted to about 4.4 with 6N HCl. The suspension obtained is filtered and washed on the filter with 60 ml of acetone / water 1/1 and with 60 ml of Acetone. The solid is dried under vacuum at 50 ° C to obtain 13.58 g of the product (2) as an off-white solid (quantitative yield).

### EXAMPLE 5

### Preparation acid 4-(2-(2-amino-4,7-dihydro-4-oxo-3H-pyrrolo(2,3-d) pyrimidin-5-yl) ethyl) benzoic sodium salt, sodium salt of compound (2) Scheme I

2 M NaOH (pH about 13) are added until complete dissolution to a suspension of 10.0 g of compound (2) from the example 4 in 60 ml of water. The pH is then adjusted to 9 with 1M HCl thus obtaining an abundant precipitation of the product. After 1 hour of stirring at room temperature, filtration and drying under vacuum at 50°C the sodium salt of (2) as a white solid was obtained.

### EXAMPLE 6

### Preparation of diethyl N-(4-(2-(2-amino-4,7-dihydro-4-oxo-3H-pyrrolo(2,3-d) pyrimidin-5-yl) ethyl) benzoyl-L-glutamate, compound (3) of Scheme I.

36 ml of 1 M NaOH, 60 ml of water and 64 ml of isopropanol are added to 10.72 g of acid (2) from Example 4 at room temperature. After 60 minutes of stirring at room temperature 5.93 ml of N-methyl morpholine (NMM), 64 ml of isopropanol and 9:47 g of 2-chloro-4,6-dimethoxy-1,3,5-triazine (CDMT) are added to the mixture. The mixture is kept under stirring at room temperature for 20 minutes and is then added with 2.18 ml of NMM and 4.74 g of diethyl-L-glutamate hydrochloride. After 40 minutes a further addition of 2.18 ml of NMM and 4.74 g of diethyl-L-glutamate hydrochloride is made. After 6 hours of reaction 192 ml of water are added to the mixture, the suspension thus obtained is kept under stirring overnight, filtered and the solid washed with water (3x 30 ml) and dried under vacuum at 50°C 15.76 g of diethyl ester of pemetrexed (3) as a cream-colored solid (yield 90.7%) are obtained.
Analytic data: HPLC purity 98.9%

The product has the X-ray diffraction spectrum shown in Figure 1 and corresponds to a partially amorphous product.

Depending on the different batches of reaction compounds in lesser degree of crystallinity can be obtained.

### EXAMPLE 7

### Preparation of diethyl N-(4-(2-(2-amino-4 ,7-dihydro-4-oxo-3H-pyrrolo (2,3-d) pyrimidin-5-yl) ethyl) benzoyl-L-glutamate, compound (3) of Schema I.

10.00 g of acid (2) from the example 4 are added at room temperature to 90 ml of ethanol, 40 ml of water and 8.83 g of 2-chloro-4,6-dimethoxy-1,3,5-triazine (CDMT).

A mixture consisting of 21.1 g NaOH, 20 ml of water and 10.3 ml of N-methyl morpholine was first added to the suspension obtained and then 10,45 g of diethyl-L-glutamate hydrochloride.

The reaction mixture is kept under stirring at 40°C for 3 hours and is filtered on a bed consisting of 0,48 g of activated carbon and 0.48 g of Celite. After washing the filter with 20 ml of ethanol/water mixture 3/2, the filtrate is heated to 40°C and added to 49 ml of water and 55 ml of Acetone.

The mixture is then cooled to room temperature and after cooling to 0°C for 1 hour, the suspension obtained is filtered, the solid washed with 80 ml of water/acetone mixture 3/1 and with 80 ml of Acetone. The solid is then dried under vacuum at 50°C. 14.25 g of diethyl ester of pemetrexed (3) as a cream-colored solid (yield 93.5%) are obtained.
pf (Hot plate): 171 °C (with a different batch a p.f. of 138°C was obtained)
X-ray diffraction spectrum: shown in Figure II
DSC shown in Figure III

### EXAMPLE 8

### Preparation of diethyl N-(4-(2-(2-amino-4,7-dihydro-4-oxo-3H-pyrrolo (2,3-d) pyrimidin-5-yl) ethyl) benzoyl-L-glutamate, compound (3) of Scheme I.

10.0 g of acid (2) from the example 4 are added at room temperature to 40 ml of isopropanol and 60 ml of NaOH 0.50 M. After 10 minutes of stirring at room temperature 25 ml of isopropanol, 5.15 ml of N-methylmorpholine (NMM), 8.83 g of CDMT and again 25 ml of isopropanol are added to the mixture. After 5 minutes 4.80 ml of N-Methylmorpholine are added followed by 10,45 g of H-Glu (OEt)₂.HCl.

The reaction mixture is then heated at 40°C for 1 hour, then 30 ml of water are added. The solution is cooled to room temperature and then added with 180 ml of water and kept under stirring at room temperature for 1 hour. The suspension is filtered, washed with water/iPrOH = 3/1 (3x30 ml), water (3 x 40 ml) and the solid dried under vacuum at 50°C. 14.52 g of diethyl ester of pemetrexed (3) as a cream-colored solid (yield 89.1%) are obtained.
Analytic data: HPLC purity 98.4%

### EXAMPLE 9

### Preparation of diethyl N-(4-(2-(2-amino-4,7-dihydro-4-oxo-3H-pyrrolo(2,3-d) pyrimidin-5-yl) ethyl) benzoyl-L-glutamate, compound (3) of Schema I.

10.0 g of acid (2) from the example 4 are added at room temperature to 40 ml of isopropanol and 60 ml of NaOH 0.50 M. After 10 minutes of stirring at room temperature 25 ml of isopropanol, 5.15 ml of N-methylmorpholine (NMM), 8.83 g of CDMT and again 25 ml of isopropanol are added to the mixture. After 5 minutes 4.80 ml of N-Methylmorpholine are added followed by 10.45 g of H-Glu (OEt) ₂.HCl.

The reaction mixture is then heated at 40°C for 1 hour, then 80 ml of water and 80 ml of acetone are added. The solution is cooled to room temperature and then kept at 0°C for 2 hours. The suspension is filtered, washed with water/acetone=3/1 (40 ml), water (3 x 40 ml) and acetone (2 x 20 ml) and the solid dried under vacuum at 50°C. 13.90 g of diethyl ester of pemetrexed (3) as a cream-colored solid (yield 85.7%) are obtained
Analytic data: HPLC purity 98.5%

### EXAMPLE 10

### Preparation of diethyl N-(4-(2-(2-amino-4,7-dihydro-4-oxo-3H-pyrrolo(2,3-d) pyrimidin-5-yl) ethyl) benzoyl-L-glutamate, compound (3) of Scheme I.

10.0 g of acid (2) from the example 4 are added at room temperature to 60 ml of isopropanol, 60 ml of water and 13.65 ml of NMM. The mixture is heated to 40°C and after 40 minutes 8.83 g of CDMT, 30 ml of isopropanol, 10:45 g of H-Glu (OEt) ₂.HCl are added. The reaction mixture is heated to 40°C for 2 hours, then 80 ml of water and 50 ml of Acetone are added. The mixture is cooled to room temperature and then maintained for 2 hours at 0°C. The suspension is filtered, washed with water/acetone = 3/1 (40 ml), water (3 x 40 ml) and acetone (2 x 20 ml) and the solid dried under vacuum at 50°C. 13.58 g of diethyl ester of pemetrexed (3) as a cream-colored solid (yield 83.8%) are obtained.
Analytic data: HPLC purity 98.7%

### EXAMPLE 11

### Trituration/crystallization with 2-methyl tetrahydrofuran/Absolute ethanol 10/1 of diethyl N-(4-(2-(2-amino-4,7-dihydro-4-oxo-3H-pyrrolo(2,3-d) pyrimidin -5-yl) ethyl) benzoyl-L-glutamate, compound (3) of Scheme I.

10:36 g of crude pemetrexed diethyl ester (3) are shredded / crystallized to weak reflux in 62 ml of a mixture of 2-Me THF / absolute ethanol 10/1. The solution is slowly cooled to room temperature and maintained at 0°C for 5 hours. The suspension is then filtered and the solid washed with 10 ml of 2-Me THF cooled to 0°C. After drying under vacuum at 40°C 11.8 g of product 3 as a cream solid (yield 78.3%) were obtained.
Analytic data: HPLC purity 99.9%
X-ray diffraction spectrum: shown in Figure IV

### EXAMPLE 12

### Crystallization with Methyl ethyl ketone/Ethanol 96% 9/2 of diethyl N-(4-(2-(2-amino-4,7-dihydro-4-oxo-3H-pyrrolo(2,3-d)pyrimidin-5-yl)ethyl) benzoyl-L-glutamate, compound (3) of Scheme I.

14.00 g of crude pemetrexed diethyl ester (3) are dissolved with stirring at weak reflux with 182 ml of mixture Metyethylketone/EtOH 96% 9/2. The obtained solution is allowed to cool at room temperature, the suspension is cooled at 0°C for 2 hours, filtered and the solid washed with 84 ml of methyl ethyl ketone. After drying under vacuum at 50°C 13.24 g of product 3 as a cream solid (yield 94.5%) were obtained.
Analysis: HPLC purity 99.83%
p.f. (Hot Stage): 175°C (pf varies slightly depending on the batch)
X-ray diffraction spectrum: shown in Figure V
SDC is shown in Figure VI

An analogous compound can be obtained by crystallization from the following solvents: ethanol, ethyl acetate/ethanol, acetonitrile

### EXAMPLE 13 (reference example)

### Preparation of p-toluenesulfonate of diethyl N-(4-(2-(2-amino-4,7-dihydro-4-oxo-3H-pyrrolo(2,3-d) pyrimidin-5-yl)ethyl)benzoyl-L-glutamate, compound (4) of Scheme I.

11.4 g of the product (3) of Example 5 are suspended in 142 ml of absolute ethanol. The mixture is heated to 50°C and the solution thus obtained is added to a solution of 11.2 g of p-toluenesulfonic acid monohydrate (pTsOH.H2O) in 142 ml of absolute ethanol. The suspension obtained is heated at 80°C for 2 hours, then cooled to room temperature and filtered. The wet solid is triturated at reflux for 1 hour with 242 ml of absolute ethanol.

The suspension is filtered and the solid dried under vacuum at 50°C. The light pink product is heated to reflux in 65 ml of DMSO and 260 ml of absolute ethanol for 1.5 hours. After cooling at 10°C the suspension is filtered and the wet solid is triturated at 5°C with 250 ml of absolute ethanol for 30'. The solid is dried under vacuum at 50°C to give 12.2 g of a light pink solid (Yield 79%).
Analytic data: HPLC purity 99.2%

### EXAMPLE 14

### Preparation of pemetrexed, compound (5)

A suspension of 9.60 g of the product (3) obtained in Example 6 (or alternatively one of Examples 7-12) in 62 ml of 1 M NaOH is kept under stirring at room temperature for about 1 hour. After addition of 75 ml of ethanol, the solution is acidified to pH 3 with 4M HCl and the mixture is heated to 70-75°C for about 20'. The suspension obtained is cooled at room temperature and filtered. The wet solid is triturated with 93 ml of ethanol/water 1/1 at room temperature for 2 hours. After filtration, the solid is dried under vacuum at 50°C to give 6.15 g of off-white solid (98% yield).
Analytic data: HPLC purity 99.8%

### EXAMPLE 15

### Preparation of pemetrexed hemipentahydrate disodium salt, compound (6).

To a suspension of 7.0 g of Pemetrexed 5 in 38 ml of water, 1 M NaOH is added up to a pH of about 8 (pH range from 7.5 to 8.5). The solution is heated to 70°C and added to 398 ml of ethanol. The suspension is allowed to cool at room temperature, filtered and the solid is dried at 50°C.

7.4 g of disodium hemipentahydrate of pemetrexed as a white solid (yield 88%) are obtained
Analytic data: HPLC purity 99.8%
EXAMPLE 16

### Preparation of pemetrexed hemipentahydrate disodium salt, compound (6)

To a suspension of 10.0 g of pemetrexed ester (3) as obtained in Example 6 (or alternatively one of Examples 7-12), 100 ml of 1M NaOH are added and the mixture is stirred at room temperature until complete dissolution of the solid. After addition of 60 ml of ethanol is added slowly a solution of 1 M HCl up to a pH of about 8 (pH range from 7.5 to 8.5). The mixture is heated to 55°C and added to 160 ml of ethanol. The suspension is allowed to cool at room temperature, filtered and the solid is dried at 50°C.

9.6 g of hemipentahydrate disodium of Pemetrexed as a white solid (yield 90%) are obtained. Analytic data: HPLC purity 99.8%

### EXAMPLE 17

### Preparation of pemetrexed lysine salt, compound (1)

8.8 g of Pemetrexed (5) are added to a solution of 9 g of lysine hydrate in 41 ml of water. The solution is heated to 70°C and added to 249 ml of ethanol. The suspension is allowed to cool to room temperature, filtered and the solid is dried at 50°C.

13.8 g of pemetrexed lysine salt as a white solid are obtained. (Yield 93%).
Analytic data: HPLC purity 99.93%, lysine content (HPLC): 39.5%
Weight loss (TGA): 3.8%
X-ray diffraction spectrum: shown in Figure VII and VIII
DSC, shown in Fig IX: p.f. 230 ° C (dec)

¹H-NMR (D2O): δ 1.46 (m, 2H), 1.52 (m, 2H), 1.73 (m, 4H), 1.92 (m, 4H), 2.9 (m, 1 H), 2.22 (m, 1 H), 2.37 (m, 2H), 2.81 (s, 4H), 3.03 (t, 4H), 3.78 (t, 2H), 4:37 (dd, 1H), 6.34 (s, 1H), 7.19 (d, 2H), 7.68 (d, 2H)

13C_NMR (D 2 O): δ 24.2, 29.2, 29.6, 31.2, 32.7, 37.1, 38.2, 41.8, 57.3, 58.8, 101.5, 118.3, 120.9, 129.8, 131.3, 133.4, 149.5, 153.5, 154.8, 163.9, 172.8, 177.4, 181.8, 185.0.

### EXAMPLE 18

Stability studies of salt of pemetrexed (bi)lysine disodium salt.

Stability studies at 25°C ± 5°C, 45°C ± 5°C and 100°C were carried out on the salts thereof obtained according to the procedure reported in Examples 15, 16, 17 and, for comparison, on the heptahydrate sodium salt prepared according to procedure EP 1259513.

The samples were packed in glass containers in air or alternatively in a controlled atmosphere.

The purity of the samples was determined at varying time intervals (1, 3, 6 months for samples stored at 25°C ± 5°C and 45°C ± 5°C, 6 days for samples stored at 100°C) by HPLC analysis using the following procedure:
Apparatus high pressure liquid chromatograph with a UV detector with variable wavelength Agilent 1100 or equivalent
Column: Sunfire C18, 3.5 µm, 100x4.6 mm
Operating conditions:
   Mobile phase A: 0.1 % H₃PO₄
   Mobile phase B = CH₃CN
Gradient elution; t = 0, A/B 88/12, t = 10 min, A/B 88/12, t = 30min, A/B 30/7
Flow rate: 1.5 ml / min Detector: λ = 230 nm, injection volume: 20 I, T: 35°C White: Water

### EXAMPLE 19

### Preparation of a pharmaceutical composition with the pemetrexed lysine salt.

In a suitable reactor in an inert atmosphere pemetrexed disodium (6), mannitol and water (water for injection-WFI) are charged in order to have a solution consisting of 25 mg/ml of pemetrexed acid, 25 mg/ml of mannitol. The pH of the solution obtained is adjusted to 7.2 ± 0.6 with 0.1 N NaOH or HCl 0.1 N. The solution is then filtered twice on 0,22 µm, added to vials, in the required quantity and lyophilized.

### EXAMPLE 20

### Preparation of a pharmaceutical composition with the pemetrexed lysine salt.

In a suitable reactor in an inert atmosphere the pemetrexed lysine salt (1), mannitol and water (water for injection-WFI) are charged in order to have a solution consisting of 25 mg/ml of pemetrexed acid, 25 mg/ml of mannitol. The pH of the solution obtained is adjusted to 7.2 ± 0.6 with 0.1 N NaOH or HCl 0.1 N. The solution is then filtered twice on 0,22 µm, added to in vials, in the required quantity and lyophilized.

## Claims

1. A process for the preparation of a pemetrexed salt having general formula I 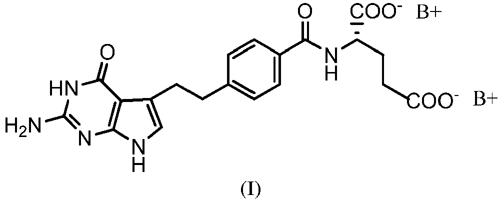 wherein B+ represents Na⁺ or protonated lysine
comprising the following steps:
a) reacting 4-(2-(2-amino-4,7-dihydro-4-oxo-3H-pyrrol(2,3-d) pyrimidin-5-yl) ethyl) benzoic acid (2) with the L diethyl glutamate hydrochloride in presence of a coupling reagent to obtain the pemetrexed diethyl ester (3) 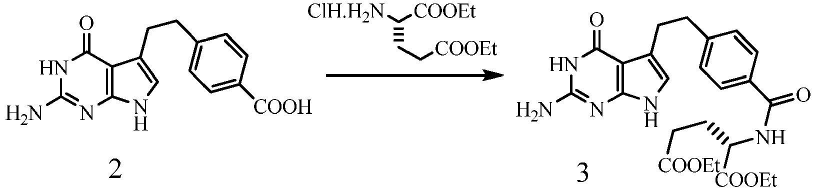
b) hydrolyzing the pemetrexed diethyl ester (3) to pemetrexed acid (5) 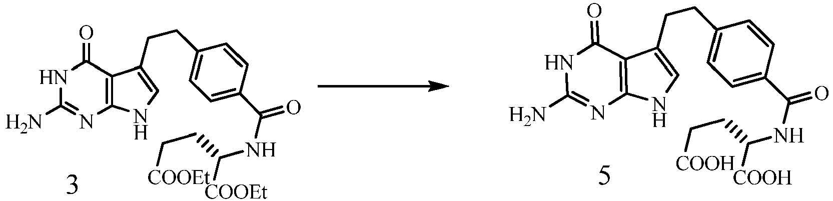
c) salifying with a base selected from NaOH or lysine to obtain the corresponding pemetrexed salt
**characterized in that** in step a):
i) the acid (2) is previously converted *in situ* to the corresponding sodium carboxylate or morpholine carboxylate which may be isolated or directly reacted;
ii) the reaction solvent used consists of a hydro-alcoholic mixture of solvents wherein the alcohol is selected from the group: methanol, ethanol, isopropanol, n-propanol;
iii) the intermediate pemetrexed diethyl ester (3) directly precipitates from the reaction mixture with a purity >98%.

2. The process according to claim 1, wherein said hydro-alcoholic mixture of solvents is represented by water mixed with isopropyl alcohol or ethyl alcohol.

3. The process according to claim 2, wherein the components of said mixture water/isopropyl alcohol or ethyl alcohol in step a) are in a ratio of 3/1 to 1/3 each other.

4. The process according to any one of claims 1 to 3, wherein the coupling reagent in step a) is selected from the group: CDMT (2-chloro-4,6-dimethoxy-1,3,5-triazine), EDC (1-ethyl-3(3-dimethylaminopropyl) carbodiimide and DMTMM (4-(4,6-dimethoxy-1,3,5-triazin-2-yl)-4-methylmorpholine chloride), preferably CDMT.

5. The process according to any one of claims 1 to 4, wherein the reaction in step a) is carried out at a temperature between 0°C and 50°C or between 15°C and 50°C.

6. The process according to any one of claims 1 to 5, wherein the lysine salt is obtained by addition to pemetrexed acid (5) of an aqueous solution containing from 2 to 4 equivalents of hydrate lysine, preferably 3 equivalents.

7. The process according to any one of claims 1 to 6, wherein in step c) the lysine salt formed in solution is precipitated by addition of an organic solvent miscible with water selected from the group: acetone, ethanol, methanol or isopropanol, preferably ethanol.

8. The process according to any one of claims 1-7 wherein, as an alternative to synthetic steps b) and c) it is possible to obtain the pemetrexed disodium salt by direct hydrolysis of the diester (3) without isolation of the intermediate pemetrexed acid (5).

9. The process according to any one of claims 1 to 8, wherein the pemetrexed diethyl ester (3) is used directly in next step without any further purification.

10. A compound of formula (1), acid-N-[4-[2-(2-amino-4-oxo-4,7-dihydro-3H-pyrrolo [2,3-d] pyrimidin-5-yl) ethyl] benzoyl]-L-glutamic (pemetrexed) salt of (bi)lysine 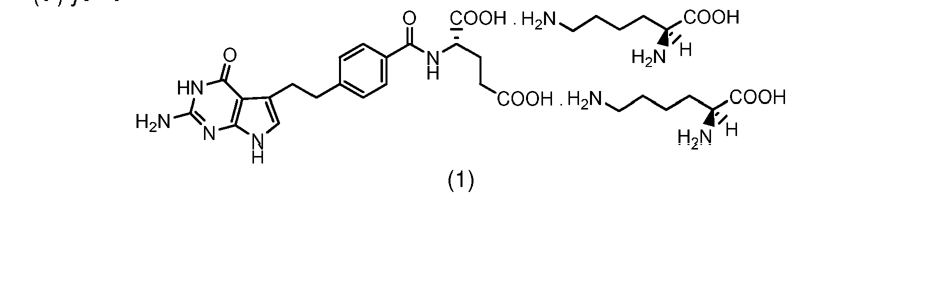 in an anhydrous or hydrate form.

11. The compound according to claim 10, having a percentage of water comprised in the range of about 0-22% by weight.

12. A process for the synthesis of the compound according to claim 10, comprising a step of salification of pemetrexed (5) by protonated lysine in accordance with claim 1.

13. The compound according to any one of claims 10-11, for use as medicament.

14. The compound according to claims 10-11 for use according to claim 13, characteized in that it is used as anti-cancer medicament.

15. A pharmaceutical composition comprising the compound according to any one of claims 10 to 11 and at least one pharmacologically acceptable excipient.

16. A pharmaceutical composition according to claim 15 for use as anti-cancer composition.

## Patentansprüche

1. Ein Verfahren zur Herstellung eines Pemetrexedsalzes, welches die allgemeine Formel 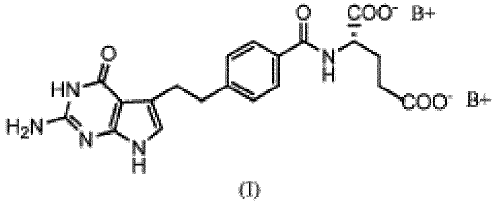 aufweist,
wobei B+ für Na⁺ oder protoniertes Lysin steht,
umfassend die folgenden Schritte:
a) Umsetzen von 4-(2-(2-Amino-4,7-dihydro-4-oxo-3H-pyrrol(2,3-d)pyrimidin-5-yl)ethyl)benzoesäure (2) mit dem L-Diethylglutamat-Hydrochlorid in Gegenwart eines Kupplungsreagens, um den Pemetrexed-Diethylester (3) zu erhalten 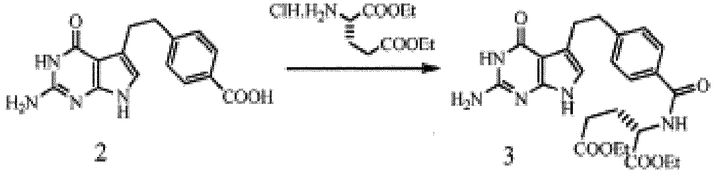
b) Hydrolysieren des Pemetrexed-Diethylesters (3) zu Pemetrexedsäure (5) 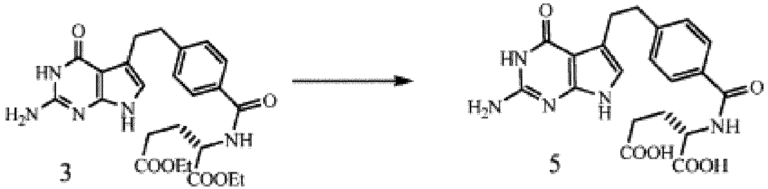
c) Salzbildung mit einer Base, ausgewählt aus NaOH oder Lysin, um das entsprechende Pemetrexedsalz zu erhalten
**dadurch gekennzeichnet, dass** in Schritt a):
i) die Säure (2) vorher *in situ* in das entsprechende Natriumcarboxylat oder Morpholincarboxylat umgewandelt wird, welches isoliert oder direkt umgesetzt werden kann;
ii) das verwendete Reaktionslösungsmittel aus einem hydroalkoholischen Gemisch aus Lösungsmitteln besteht, wobei der Alkohol ausgewählt ist aus der Gruppe: Methanol, Ethanol, Isopropanol, n-Propanol;
iii) das Zwischenprodukt Pemetrexed-Diethylester (3) direkt mit einer Reinheit von >98% aus dem Reaktionsgemisch ausfällt.

2. Das Verfahren gemäß Anspruch 1, wobei das hydroalkoholische Gemisch aus Lösungsmitteln durch mit Isopropylalkohol oder Ethylalkohol gemischtes Wasser dargestellt ist.

3. Das Verfahren gemäß Anspruch 2, wobei die Komponenten des Gemischs Wasser/Isopropylalkohol oder Ethylalkohol in Schritt a) in einem Verhältnis von 3/1 bis 1/3 zueinander vorliegen.

4. Das Verfahren gemäß einem der Ansprüche 1 bis 3, wobei das Kupplungsreagens in Schritt a) ausgewählt ist aus der Gruppe: CDMT (2-Chlor-4,6-dimethoxy-1,3,5-triazin), EDC (1-Ethyl-3-(3-dimethylaminopropyl)carbodiimid und DMTMM (4-(4,6-Dimethoxy-1,3,5-triazin-2-yl)-4-methylmorpholinchlorid), vorzugsweise CDMT.

5. Das Verfahren gemäß einem der Ansprüche 1 bis 4, wobei die Umsetzung in Schritt a) bei einer Temperatur zwischen 0°C und 50°C oder zwischen 15°C und 50°C durchgeführt wird.

6. Das Verfahren gemäß einem der Ansprüche 1 bis 5, wobei das Lysinsalz durch Zugabe einer wässrigen Lösung enthaltend 2 bis 4 Äquivalente von Hydratlysin, vorzugsweise 3 Äquivalente, zu der Pemetrexedsäure (5) erhalten wird.

7. Das Verfahren gemäß einem der Ansprüche 1 bis 6, wobei in Schritt c) das in Lösung gebildete Lysinsalz durch Zugabe eines organischen Lösungsmittels, welches mit Wasser mischbar ist, ausgewählt aus der Gruppe: Aceton, Ethanol, Methanol oder Isopropanol, vorzugsweise Ethanol, ausfällt.

8. Das Verfahren gemäß einem der Ansprüche 1-7, wobei, als eine Alternative zu den Syntheseschritten b) und c), es möglich ist, das Pemetrexed-Dinatriumsalz durch direkte Hydrolyse des Diesters (3) ohne Isolierung des Zwischenprodukts Pemetrexedsäure (5) zu erhalten.

9. Das Verfahren gemäß einem der Ansprüche 1 bis 8, wobei der Pemetrexed-Diethylester (3) im nächsten Schritt direkt ohne weitere Reinigung verwendet wird.

10. Eine Verbindung der Formel (1), Säure-N-[4-[2-(2-amino-4-oxo-4,7-dihydro-3H-pyrrolo[2,3-d]pyrimidin-5-yl)ethyl]benzoyl]-L-glutamin(Pemetrexed)salz von (Bi)lysin 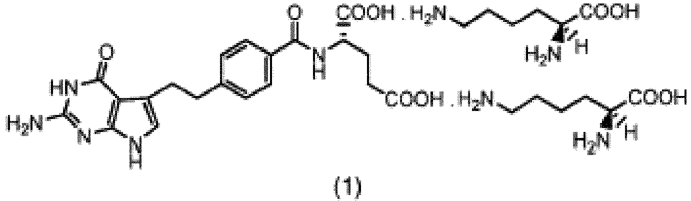 in einer wasserfreien Form oder Hydratform.

11. Die Verbindung gemäß Anspruch 10, welche einen Anteil an Wasser aufweist, der im Bereich von etwa 0-22 Gew.-% umfasst ist.

12. Ein Verfahren zur Synthese der Verbindung gemäß Anspruch 10, umfassend einen Schritt der Salzbildung von Pemetrexed (5) durch protoniertes Lysin gemäß Anspruch 1.

13. Die Verbindung gemäß einem der Ansprüche 10-11 zur Verwendung als Medikament.

14. Die Verbindung gemäß den Ansprüchen 10-11 zur Verwendung gemäß Anspruch 13, **dadurch gekennzeichnet, dass** es als Anti-Krebs-Medikament verwendet wird.

15. Ein Arzneimittel, umfassend die Verbindung gemäß einem der Ansprüche 10 bis 11 und mindestens einen pharmakologisch verträglichen Exzipienten.

16. Ein Arzneimittel gemäß Anspruch 15 zur Verwendung als Anti-Krebs-Zusammensetzung.

## Revendications

1. Procédé pour la préparation d'un sel de pémétrexed ayant pour formule générale 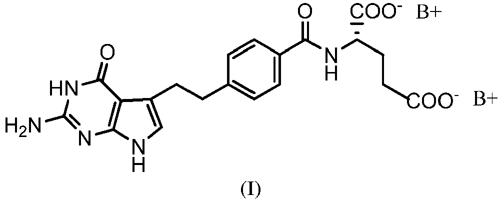 dans laquelle B+ représente Na⁺ ou une lysine protonée
comprenant les étapes suivantes consistant à :
a) faire réagir l'acide 4-(2-(2-amino-4,7-dihydro-4-oxo-3H-pyrrol(2,3-d)pyrimidin-5-yl)éthyl)benzoïque (2) avec du chlorhydrate de L-diéthylglutamate en présence d'un réactif de couplage pour obtenir l'ester diéthylique de pémétrexed (3) 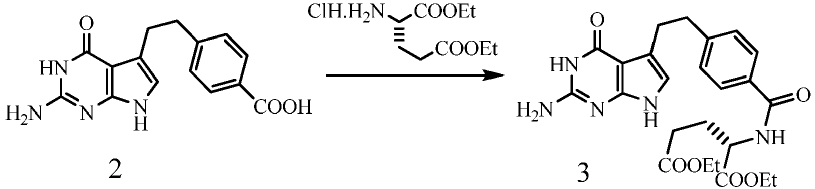
b) hydrolyser l'ester diéthylique de pémétrexed (3) pour donner l'acide de pémétrexed (5) 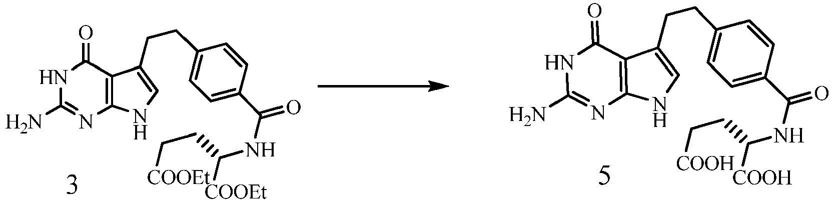
c) salifier avec une base choisie parmi NaOH ou la lysine pour obtenir le sel de pémétrexed correspondant
**caractérisé en ce que**, à l'étape a) :
i) l'acide (2) est converti au préalable *in situ* en le carboxylate de sodium ou carboxylate de morpholine correspondant qui peut être isolé ou que l'on peut faire réagir directement ;
ii) le solvant réactionnel utilisé se compose d'un mélange hydro-alcoolique de solvants, dans lequel l'alcool est choisi dans le groupe constitué par : le méthanol, l'éthanol, l'isopropanol, le n-propanol ;
iii) l'ester diéthylique de pémétrexed intermédiaire (3) se précipite directement à partir du mélange réactionnel en une pureté > 98 %.

2. Procédé selon la revendication 1, dans lequel ledit mélange hydro-alcoolique de solvants est représenté par de l'eau mélangée à de l'alcool isopropylique ou de l'alcool éthylique.

3. Procédé selon la revendication 2, dans lequel les composants dudit mélange eau/alcool isopropylique ou alcool éthylique de l'étape a) sont présents en un rapport de 3/1 à 1/3 les uns sur les autres.

4. Procédé selon l'une quelconque des revendications 1 à 3, dans lequel le réactif de couplage de l'étape a) est choisi dans le groupe constitué par: la CDMT (2-chloro-4,6-diméthoxy-1,3,5-triazine), l'EDC (1-éthyl-3-(3-diméthylamino-propyl)carbodiimide et le DMTMM (chlorure de 4-(4,6-diméthoxy-1,3,5-triazin-2-yl)-4-méthylmorpholine), de préférence la CDMT.

5. Procédé selon l'une quelconque des revendications 1 à 4, dans lequel la réaction de l'étape a) est réalisée à une température située entre 0 °C et 50 °C ou entre 15 °C et 50 °C.

6. Procédé selon l'une quelconque des revendications 1 à 5, dans lequel le sel de lysine est obtenu par l'addition à l'acide de pémétrexed (5) d'une solution aqueuse contenant de 2 à 4 équivalents d'hydrate de lysine, de préférence 3 équivalents.

7. Procédé selon l'une quelconque des revendications 1 à 6, dans lequel, à l'étape c), le sel de lysine formé en solution est précipité par l'addition d'un solvant organique miscible avec l'eau choisi dans le groupe constitué par : l'acétone, l'éthanol, le méthanol ou l'isopropanol, de préférence l'éthanol.

8. Procédé selon l'une quelconque des revendications 1 à 7, dans lequel, en variante aux étapes de synthèse b) et c), il est possible d'obtenir le sel disodique de pémétrexed par hydrolyse directe du diester (3) sans isoler l'acide de pémétrexed intermédiaire (5).

9. Procédé selon l'une quelconque des revendications 1 à 8, dans lequel l'ester diéthylique de pémétrexed (3) est utilisé directement au cours de la prochaine étape sans purification ultérieure quelconque.

10. Composé de formule (1), acide N-[4-[2-(2-amino-4-oxo-4,7-dihydro-3H-pyrrolo-[2,3-d]-pyrimidin-5-yl)éthyl]-benzoyl]-L-glutamique (pémétrexed) sel de (bi)lysine, 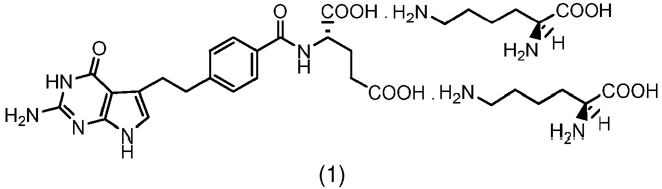 sous forme anhydre ou hydratée.

11. Composé selon la revendication 10, ayant un pourcentage d'eau compris dans la plage d'environ 0 à 22 % en poids.

12. Procédé pour la synthèse du composé selon la revendication 10, comprenant une étape de salification du pémétrexed (5) par la lysine protonée selon la revendication 1.

13. Composé selon l'une quelconque des revendications 10 à 11, pour l'utilisation comme médicament.

14. Composé selon les revendications 10 à 11 pour une utilisation selon la revendication 13, **caractérisé en ce qu'**il est utilisé comme médicament anti-cancéreux.

15. Composition pharmaceutique comprenant le composé selon l'une quelconque des revendications 10 à 11 et au moins un excipient pharmaceutiquement acceptable.

16. Composition pharmaceutique selon la revendication 15 pour une utilisation comme composition anti-cancéreuse.
